(19) Europäisches Patentamt
European Patent Office
Office européen des brevets

(11) EP 0 610 140 B1

(12) FASCICULE DE BREVET EUROPEEN

(45) Date de publication et mention de la délivrance du brevet:
**12.01.2000 Bulletin 2000/02**

(51) Int. Cl.[7]: **C07D 403/12**, A61K 31/505

(21) Numéro de dépôt: **94400238.5**

(22) Date de dépôt: **04.02.1994**

# (54) Dérivés de 2-[4-(4-azolylbutyl)-1-pipérazinyl]-5-hydroxypyrimidine, leur préparation et leur application en tant que médicaments

2-[4-(4-Azolylbutyl)-1-piperazinyl]-5-hydroxypyrimidin-Derivate, deren Herstellung und deren Verwendung als Heilmittel

2-[4-(4-Azolylbutyl)-1-piperazinyl]-5-hydroxypyrimidin derivatives, their preparation and their use as medicines

(84) Etats contractants désignés:
**AT BE CH DE DK FR GB GR IE IT LI LU MC NL PT SE**

(30) Priorité: **05.02.1993 FR 9301293**

(43) Date de publication de la demande:
**10.08.1994 Bulletin 1994/32**

(73) Titulaire:
**LABORATORIOS DEL DR. ESTEVE, S.A.
08026 Barcelona (ES)**

(72) Inventeurs:
- **Frigola-Constansa, Jordi
E-08013 Barcelone (ES)**
- **Merce-Vidal, Ramon
E-08014 Barcelone (ES)**

(74) Mandataire: **Warcoin, Jacques
Cabinet Régimbeau,
26, avenue Kléber
75116 Paris (FR)**

(56) Documents cités:
**EP-A- 0 129 128**
**EP-A- 0 382 637**
**DE-A- 3 248 160**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 610 140 B1

## Description

La présente invention concerne de nouveaux dérivés de 2-[4-(4-azolylbutyl)-1-pipérazinyl]pyrimidine de formule générale I, leur procédé de préparation, leur application comme médicaments, ainsi que les compositions pharmaceutiques les comprenant,

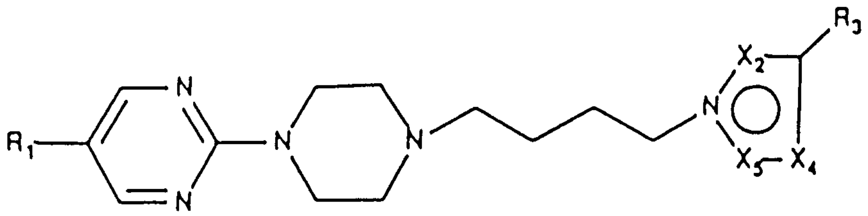

I

où $R_1$ représente un radical hydroxyle, $X_2$ représente un atome d'azote ou un groupe C-$R_2$, $X_4$ représente un atome d'azote ou un groupe C-$R_4$, $X_5$ représente un atome d'azote ou un groupe C-$R_5$, et $R_2$, $R_3$, $R_4$ et $R_5$, identiques ou différents, représentent un atome d'hydrogène, un halogène, un radical méthyl, ainsi que leurs sels physiologiquement acceptables.

Les composés objets de l'invention peuvent être utilisés dans l'industrie pharmaceutique comme produits intermédiaires de synthèse pour la préparation de médicaments.

Depuis quelques années on connaît diverses amides cycliques à activité anxiolytique dérivées d'arylpipérazines de formule générale II

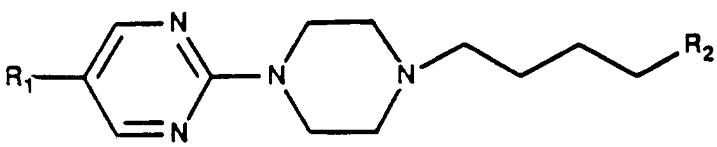

II

comme : Buspirone (Drugs of the Future, 1976, 1, 409) ; Gepirone (Drugs of the Future, 1985, 10, 456) ; Ipsapirone (Drugs of the Future, 1986, 11, 565) ; et Tandospirone (Drugs of the Future, 1989, 14, 1148), dans lesquelles $R_1$ représente un atome d'hydrogène et $R_2$ est indiqué ci-dessous pour chacune d'entre elles

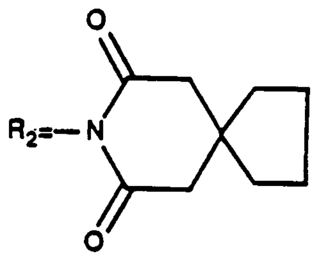

**Buspirone**

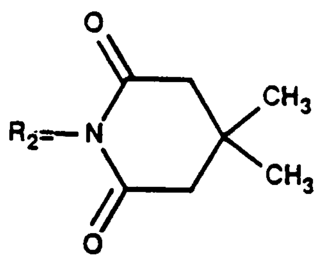

**Gepirone**

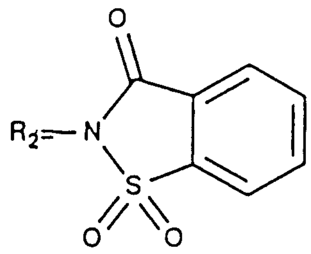

Ipsapirone

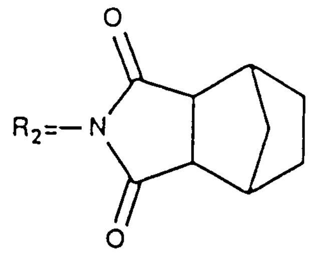

Tandospirone

Il a également été trouvé, dans notre laboratoire, une série d'aryl(ou-hétéroaryl)-pipérazinyl-alcoyl-azoles utiles comme agents non benzodiazé-piniques pour le traitement de l'anxiété (brevets européens N°EP 382637, 497659 et 502786). En outre ces composés sont également utiles pour le traitement d'autres désordres du comportement (brevets européens N° EP 429360 et 497658). Certains des composés les plus intéressants sont des azoles substitués en position 1 par un groupe pyrimidinyl-pipérazinyl-butyle de formule I dans laquelle $R_1$ représente un atome d'hydrogène et $X_2$ , $X_4$ , $X_5$ , $R_2$ , $R_3$ , $R_4$ et $R_5$ ont les significations indiquées précédemment.

Un composé qui convient particulièrement est le Lesopitron (E-4424), composé de formule générale I dans laquelle $R_1$ représente un atome d'hydrogène, $X_2$ représente un groupement $CR_2$ , $X_4$ représente un groupement$CR_4$ , $X_5$ représente un atome d'azote, $R_2$ et $R_4$ représentent un hydrogène, et $R_3$ représente un chlore. On a montré que le Lesopitron est un composé ayant un profil d'activité très intéressant et beaucoup plus puissant que la Buspirone ou l'Ipsapirone (cf B. Costall et al. J. Pharmacol.& Experimental Therapeutics, volume 262, pages 90-98, 1992.

Il a été dit que les composés de formule générale II subissent une biotransformation "in vivo" pour donner lieu fondamentalement à deux types de métabolites. L'un des métabolites est la pyrimidylpipérazine de formule III

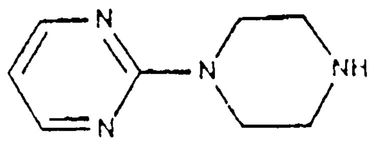

III

composé qui se révèle être actif tant d'un point de vue biochimique que pharmacologique, comme il a été décrit dans de nombreuses publications scientifiques dont on citera comme exemples les suivantes: E. Tatarczynska et al. Pol. J. Pharmacol Pharm. 1989, 41, 51; A. Diaz-Marot et al. J. Chromatogr. 1989, 490, 470; S. Caccia et al. Acta Pharm. Jugosl. 1990, 40, 441; H. Nocon et al. J. Pharm. Pharmacol. 1990, 42, 642.

Un autre métabolite des composés de formule générale II cités antérieurement est le dérivé 5-hydroxy correspondant, de formule générale II dans laquelle $R_1$ représente un radical hydroxyle et $R_2$ a les significations mentionnées plus haut. Au contraire de ce qui se produit avec le composé III, il a été clairement démontré que les composés de formule générale II dans lesquels $R_1$ représente un radical hydroxyde sont biologiquement inactifs. Ce comportement est celui que l'on observe habituellement quand on hydroxyle les composés aromatiques dans un processus métabolique (cf par exemple: "Drug Metabolism. Chemical and Biochemical Aspects" B. Testa et P. Jenner, Marcel Dekker Inc., New York 1976). Comme bibliographie relative à l'inactivité biologique des composés II dans lesquels $R_1$ représente un radical hydroxyle et$R_2$ a les significations indiquées plus haut, on signalera la suivante: R.E. Gammans, et al. The American Journal of Medicine, 1986, 80(3B), 41; C.P. Vander Maelen et al. Eur. J. Pharmacol., 1986, 129, 123; K. Ishizumi et al. Chem. Pharm. Bull., 1991, 39, 228; H.K. Jajoo, Drug Metabolism & Disposition, 1989, 17, 625; H.K. Jajoo, Drug Metabolism & Disposition, 1989, 17, 634.

On a maintenant découvert que les nouveaux dérivés de 2-[4-(4-azolylbutyl)-1-pipérazinyl]-5-hydroxypyrimidine de formule générale I dans laquelle $R_1$ représente un radical hydroxyle, et $X_2$, $X_4$ , $X_5$ , $R_2$ , $R_3$ , $R_4$ et $R_5$ ont les significations indiquées précédemment, et qui font l'objet de l'invention, sont des métabolites des composés décrits dans les brevets antérieurs des demandeurs (EP 382 637 et EP 497 659) et, de façon surprenante, présentent une activité biologique sur le système nerveux central, en particulier anxiolytique et tranquillisante, permettant de les employer en thérapeutique pour le traitement des maladies qui affectent le système nerveux central des mammifères.

Les nouveaux dérivés de formule générale I dans laquelle $R_1$ représente un groupe hydroxyle et$X_2$ , $X_4$ , $X_5$ ,

$R_2$ , $R_3$ , $R_4$ et $R_5$ ont les significations indiquées pré précédemment, peuvent être préparés, selon l'invention, par le schéma de réactions suivant:

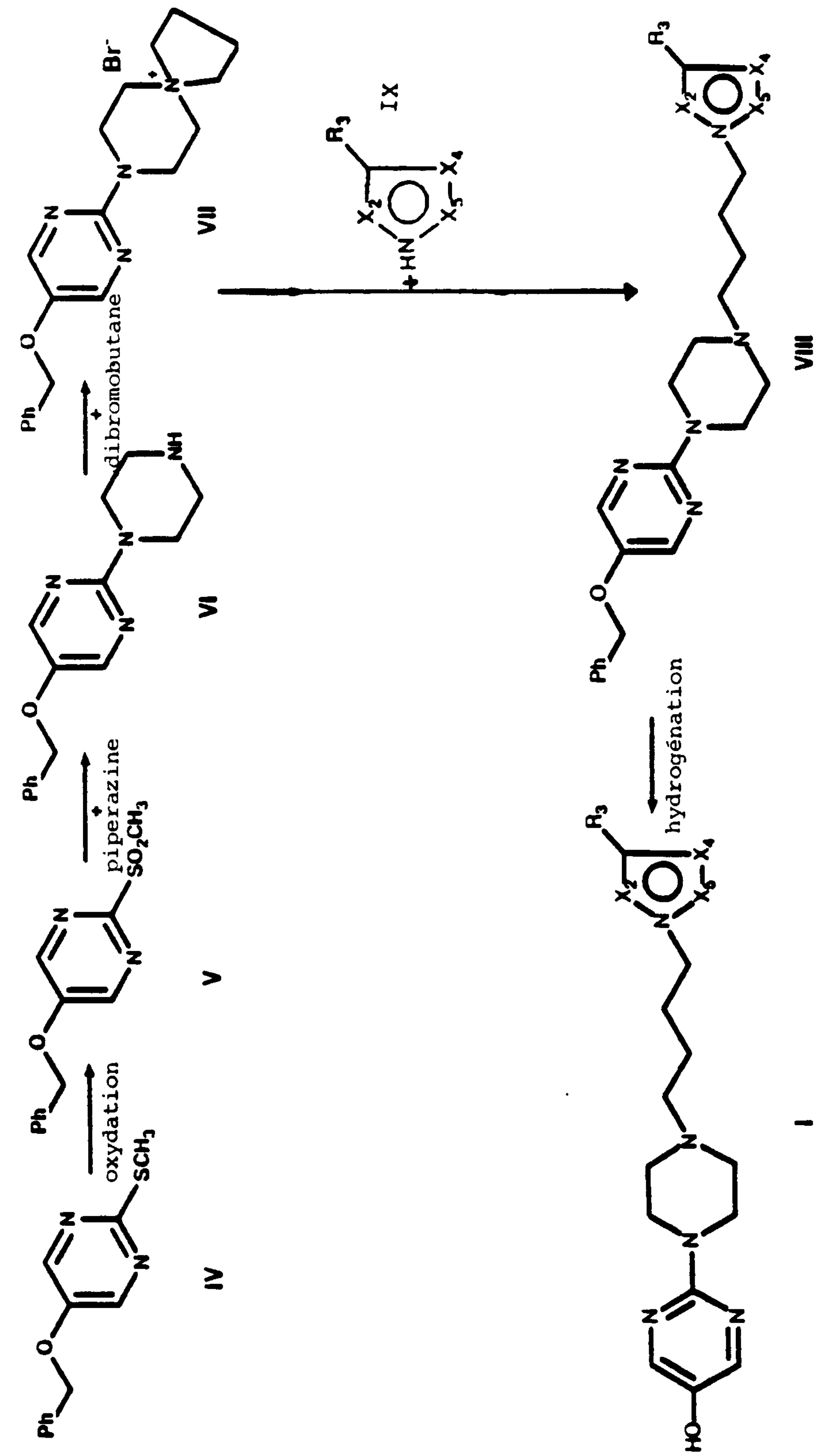

Le produit de départ est la 5-benzyloxy-2-méthylthiopyrimidine IV, que l'on peut préparer à partir du chlorure de benzyloxyacétyle, par une séquence de réactions décrites par J.H. Chesterfield et al. J. Chem. Soc., 1960, 4590 et D.T. Hurst et al. J. Chem. Soc., 1965, 7116.

Composé V. Préparation de 5-benzyloxy-2-méthylsulfonylpyrimidine

Dans une solution de 46,88 g (135,8 mmoles) d'acide m-chloroperbenzoïque à 50% dans l'eau on ajoute 15,76 g (67,9 mmoles) de 5-benzyloxy-2-méthylthiopyrimidine dans 240 ml de chloroforme. On fait refluer le mélange pendant 5 heures, on refroidit et on ajoute une solution saturée de carbonate acide de sodium. On sèche la phase organique avec du sulfate de sodium et on fait évaporer jusqu'à siccité en obtenant 17,9 g (100%) de 5-benzyloxy-2-méthylsulfonylpyrimidine.

Données spectroscopiques. IR (KBr): 1125, 1300 $cm^{-1}$.
RMN-$^1H$ ($\delta$,$CDCl_3$): 8,58 (s, 2H); 7,41 (a.c., 5H); 5,27 (s, 2H); 3,30 (s, 3H).

Composé VI. Préparation de 5-benzyloxy-2-pipérazinylpyrimidine.

On fait refluer pendant 3 heures une solution de 26,36 g (99,8 mmoles) de composé V et 85 g (99,8 mmoles) de pipérazine dans 185 ml de toluène. On laisse refroidir, on filtre et on concentre. A partir de la solution cristallisent 19,84 g (73%) de 5-benzyloxy-2-pipérazinylpyrimidine. Un échantillon recristallisé dans l'hexane a un Pf de 86-91°C.

Données spectroscopiques- IR (KBr): 1267, 144$cm^{-1}$.
RMN-$^1H$ ($\delta$, $CDCl_3$): 8,11 (s, 2H); 7,36 (a.c., 5H); 5,00 (s, 2H); 3,67 (t, 4H); 2,90 (t, 4H).

Composé VII. Préparation de bromure de 8-(5-benzyloxy-2-pyrimidinyl)-8-aza-5-azoniaspiro[4,5]décane.

On fait refluer pendant 8 heures une solution de 7,25 g (26,85 mmoles) de 5-benzyloxy-2-pipérazinyl-pyrimidine et 3,2 ml (26,85 mmoles) de 1,4-dibromobutane dans 240 ml de chloroforme et 7,42 g (53,7 mmoles) de carbonate de potassium. On fait refroidir, on filtre, on fait évaporer à siccité et on agite agvec de l'éther éthylique, d'où l'on obtient 16,0 g (91%) de bromure de 8-(5-benzyloxy-2-pyrimidinyl)-8-aza-5-azoniaspiro[4,5]décane de Pf 128-133°C.

Données spectroscopiques - IR (KBr): 1268, 1451 $cm^{-1}$.
RMN-$^1H$ ($\delta$ , $CDCl_3$) : 8,13 (s, 2H), 7,38 (a.c., 5H); 5,03 (s, 2H); 4,01 (a.c.8H); 3,77 (t, 4H); 2,35 (t, 4H).

Composés VIII- Préparation de 2-{4-[4-(azol-1-yl)-butyl]-1-pipérazinyl}-5-benzyloxypyrimidine.

Par réaction de bromure de 8-(5-benzyloxy-2-pyrimidinyl-8-aza-5-azoniaspiro[4,5]décane de formule VII avec un azole de formule générale IX

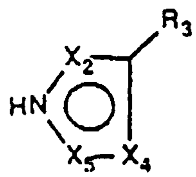

IX

dans laquelle $X_2$ représente un atome d'azote ou un groupe C-$R_2$ , $X_4$ représente un atome d'azote ou un groupe C-$R_4$ , $X_5$ représente un atome d'azote ou un groupe C-$R_5$ et $R_2$ , $R_3$ , $R_4$ et $R_5$ , identiques ou différents, représentent un atome d'hydrogène, un halogène ou un radical méthyle, on obtient un composé de formule générale VIII dans laquelle $X_2$ , $X_4$ , $X_5$ , $R_2$ , $R_3$ , $R_4$ et $R_5$ ont les significations mentionnées précédemment. La réaction s'effectue dans un solvant à point d'ébullition élevé et en présence d'une base organique ou inorganique.

Composés I- Préparation de 2-{4-[4-azol-1-yl)butyl]-1-pipérazinyl}-5-hydroxypyrimidine.

Par hydrogénation catalytique d'un composé de formule générale VIII on obtient un composé de formule générale I. Dans les deux composés, $X_2$ , $X_4$ , $X_5$ , $R_2$ , $R_3$ , $R_4$ et $R_5$ ont les significations mentionnées précédemment. La réaction s'effectue dans un solvant tel qu'un alcool, à des températures comprises entre 15 et 60°C, à des pressions

comprises entre 1 et 15 atmosphères.

Dans les exemples suivants, on indique la préparation de nouveaux dérivés selon l'invention. Les exemples qui sont présentés ci-dessous, donnés simplement à titre de précision, ne doivent cependant en aucune manière limiter la portée de l'invention.

Exemple 1- Préparation de 2-{4-[4-(4-chloro-pyrazol-1-yl)butyl]-1-pipérazinyl}-5-hydroxypyrimidine.

On maintient pendant 12 heures à reflux et on filtre à chaud un mélange de 12,55 g (30,94 mmoles) de bromure de 8-(5-benzyloxy-2-pyrimidinyl)-8-aza-5-azoniaspiro[4,5]décane, 3,48 g (34,03 moles) de 4-chloropyrazole et 8,54 g (61,89 mmoles) de carbonate de potassium dans 250 ml de diméthylformamide. On fait évaporer le diméthylformamide et on chromatographie avec du gel de silice, et on obtient 6,82 g (51,6%) de 2-{4-[4-(4-chloropyrazol-1-yl)butyl]-1-pipérazinyl}-5-benzyloxypyrimidine.

Données spectroscopiques: IR (KBr): 1256, 1270, 1363, 1447, 1482 $cm^{-1}$.
RMN-$^1H$ ($\delta$, $CDCl_3$): 8,11 (s, $H_2$); 7,40 (s, 1H); 7,38 (a.c., 5H); 7,27 (s, 1H); 5,02 (s, 2H); 4,11 (t, 2H); 3,73 (m, 4H); 2,48 (m, 4H); 2,38 (t, 2H); 1,89 (quint. 2H); 1,52 (quint., 2H).

On agite pendant 12 heures dans une atmosphère d'hydrogène à la température ambiante (24°C) et à une pression de 2 atmosphères un mélange de 6,87 g (16,07 mmoles) de 2-{4-[4-(4-chloropyrazol-1-yl)-butyl]-1-pipérazinyl}-5-benzyloxypyrimidine et 0,63 g de palladium à 10% sur charbon dans 160 ml d'éthanol. Après filtration, lavage à l'éthanol et évaporation jusqu'à siccité on chromatographie avec du gel de silice et l'on obtient 2,65 g (49%) de 2-{4-[4-(4-chloropyrazol-1-yl)butyl]-1-pipérazinyl}-5-hydroxy-pyrimidine de Pf 131-2°C.

Données spectroscopiques: IR (KBr): 1258, 1271, 1364,

RMN-$^1H$ ($\delta$, $CDCl_3$): 7,99 (s, 2H); 7,40 (s, 1H); 7,36 (s, 1H); 4,06 (t, 2H); 3,66 (t, 4H); 2,49 (t, 4H); 2,37 (t, 2H); 1,84 (quint., 2H); 1,49 (quint., 2H).

Exemple 2 - Préparation de 2-{4-[4-(4,5-dichloro-2-méthylimidazol-1-yl)butyl]-1-pipérazinyl}-5-hydroxypyrimidine.

On maintient pendant 12 heures à reflux et on filtre à chaud un mélange de 3,59 g (8,85 mmoles) de bromure de 8-(5-benzyloxy-2-pyrimidinyl)-8-aza-5-azoniaspiro[4,5]décane, 1,47 g (9,73 mmoles) de 4,5-dichloro-2-méthylimidazole et 4,89 g (35,41 mmoles) de carbonate de potassium dans 72 ml de diméthylformamide. On fait évaporer le diméthylformamide et on chromatographie avec du gel de silice, et l'on obtient 2,90 g (69%) de 2-{4-[4-(4,5-dichloro-2-méthylimidazol-1-yl)butyl]-1-pipérazinyl}-(-benzyloxypyrimidine.

Données spectroscopiques: IR (KBr): 1033, 1050, 1231, 1412, 1580 $cm^{-1}$.
RMN $^1H$ ($\delta$,$CDCl_3$): 8,11 (s, 2H); 7,37 (a.c., 5H); 5,01 (s, 2H); 3,80 (t, 2H); 3,72 (m, 4H); 2,48 (m, 4H); 2,36 (t, 2H); 2,36 (s, 3H); 1,65 (m, 4H).

On agite pendant 12 heures dans une atmosphère d'hydrogène à température ambiante (24°C) et à une pression de 0,28 MPa un mélange de 2,90 g (6,10 mmoles) de 2-{4-[4-(4,5-dichloro-2-méthylimidazol-1-yl)butyl]-1-pipérazinyl}-5-benzyloxypyrimidine et 0,30 g de palladium à 10% sur charbon dans 50 ml de méthanol. Après filtration, lavage avec du méthanol et évaporation jusqu'à siccité on chromatographie avec du gel de silice et l'on obtient 1,39 g (59%) de 2-{4-[4-(4,5-dichloro-2-méthylimidazol-1-yl)butyl]-1-pipérazinyl}-5-hydroxypyrimidine de Pf 154-6°C.

Données spectroscopiques: IR (KBr): 1250, 1277, 1355, 1408, 1429, 1445, 1469 $cm^{-1}$.
RMN-$^1H$($\delta$, $CDCl_3$): 8,00 (s, 2H); 3,84 (t, 2H); 3,65 (m, 4H); 2,49 (m, 4H); 2,39 (t, 2H); 2,32 (s, 3H); 1,69 (quint., 2H); 1,55 (quint., 2H).

Par traitement de 0,52 g (1,36 mmoles) du produit antérieur dissous dans 15 ml d'éthanol, avec de l'acide chlorhydrique à 37%, on obtient 0,43 g (75%) du monochlorhydrate de 2-{4-[4-(4,5-dichloro-2-méthylimidazol-1-yl)butyl]-1-pipérazinyl}-5-hydroxypyrimidine de Pf 212-6°C.

Données spectroscopiques: IR (KBr): 1244, 1269, 1363, 1441, 1474 $cm^{-1}$.
RMN-$^1H$ ( $\delta$, $CDCl_3$): 11,21 (a, 1H); 9,63 (s, 1H); 8,12 (s, 2H); 4,45 (m, 2H); 3,94 (m, 2H); 3,41 (m, 4H); 3,08 (m, 4H); 2,34 (s, 3H); 1,71 (m, 4H).

Activité biologique

On met en évidence l'activité biologique chez la souris, dans un essai d'exploration en chambre claire/obscure décrit par B. Costall et al., J. Pharmacology and Experimental Therapeutics, 1992, 262, 90. L'expérience s'effectue comme il est dit dans la publication mentionnée. Pour un mode opératoire plus détaillé on peut également voir B. Costall et al., Pharmacol. Biochem. Behav., 1989, 32, 777. On administre les composés par voie intrapéritonéale comme pré-traitement aigu et les résultats indiquent la dose minimale à laquelle on observe une activité significative.

| Composé | Dose minimale (mg/kg i.p.) |
|---|---|
| Diazépam | 0,125 |
| Ipsapirone | 0,5 |
| Buspirone | 0,125 |
| Lesopitron (E-4424) | 0,0001 |
| Example 1 | 0,000001 |

En tenant compte de leur forte action quant à leurs propriétés pharmacologiques, les nouveaux composés objets de l'invention peuvent être utilisés de façon satisfaisante en thérapeutique humaine et animale, en particulier dans le traitement des désordres du système nerveux central, et plus particulièrement pour le traitement de l'anxiété ou comme tranquilisants.

En thérapeutique humaine, la dose d'administration est bien entendu fonction de la gravité de la maladie. Elle pourra être comprise entre 2 et environ 20 mg par jour. Les composés objets de l'invention sont administrés sous forme de comprimés, de capsules, de solutions ou de suspensions. On indique ci-dessous, à titre d'exemple, deux formes galéniques pour les composés de l'invention.

| Exemple de formule pour comprimé | |
|---|---|
| Composé de l'exemple 1 | 5 mg |
| Lactose | 60 mg |
| Cellulose microcristalline | 25 mg |
| Povidone | 5 mg |
| Amidon prégélatinisé | 3 mg |
| Dioxyde de silicium colloïdal | 1 mg |
| Stéarate de magnésium | 1 mg |
| Poids d'un comprimé | 100 mg |
| **Exemple de formule pour capsule** | |
| Composé de l'exemple 1 | 10 mg |
| Glycéride polyoxyéthylénique | 135 mg |
| Béhénate de glycérine | 5 mg |
| Excipient: gélatine | 150 mg |

## Revendications

**1.** Dérivés de 2-[4-(4-azolylbutyl)-1-pipérazinyl]-pyrimidine caractérisés en ce qu'ils répondent à la formule générale I

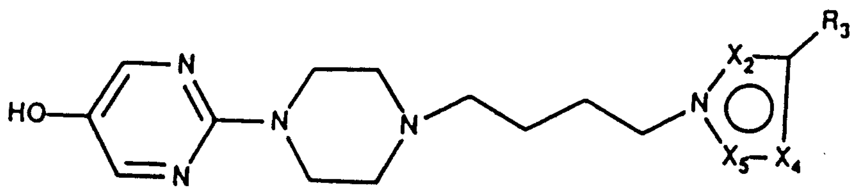

I

dans laquelle $X_2$ représente un atome d'azote ou un groupe C-$R_2$ , $X_4$ représente un atome d'azote ou un groupe C-$R_4$ , $X_5$ représente un atome d'azote ou un groupe C-$R_5$, et $R_2$ , $R_3$ , $R_4$ et $R_5$, identiques ou différents, représentent un atome d'hydrogène, un halogène, un radical méthyl, ainsi que leurs sels physiologiquement acceptables.

**2.** Composés répondant à la formule générale I selon la revendication 1, choisis parmi les suivants :

* 2-{4-[4-(4-chloropyrazol-1-yl)butyl]-1-pipérazinyl}-5-hydroxypyrimidine.
* 2-{4-[4-(4,5-dichloro-2-méthylimidazol-1-yl)-butyl]-1-pipérazinyl}-5-hydroxypyrimidine.
* chlorhydrate de 2-{4-[4-(4,5-dichloro-2-méthylimidazol-1-yl)butyl]-1-pipérazinyl}-5-hydroxypyrimidine.

**3.** Procédé de préparation des composés de formule générale I, selon les revendications 1 et 2, caractérisé en ce qu'on effectue les opérations suivantes :

* oxydation de 5-benzyloxy-2-méthylthiopyrimidine pour obtenir la sulfone correspondante de formule V,

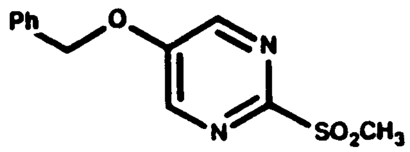

V

* remplacement du radical méthylsulfonyle par une pipérazine pour obtenir le composé de formule VI,

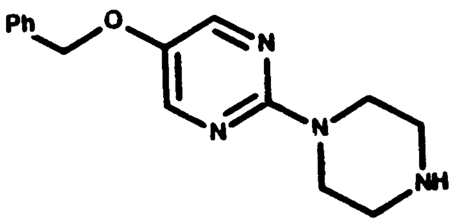

VI

* réaction du composé VI avec le dibromobutane pour obtenir le spiro correspondant de formule VII,

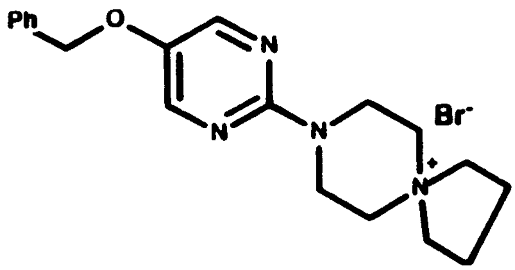

VII

* réaction du bromure de 8-(5-benzyloxy-2-pyrimidinyl)-8-aza-5-azoniaspiro [4,5] décane avec un azole de formule générale IX

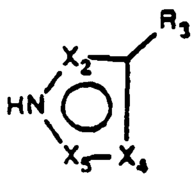

IX

dans laquelle $X_2$, $X_4$, $X_5$, $R_2$, $R_3$, $R_4$ et $R_5$ ont les significations indiquées dans la revendication 1, pour obtenir un composé de formule générale VIII

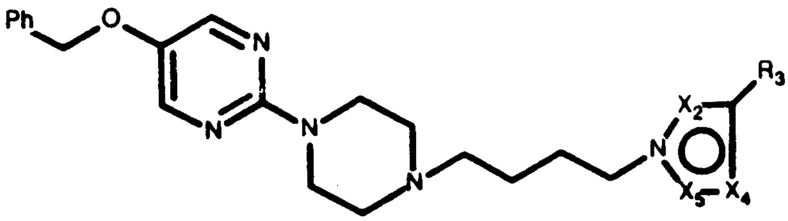

VIII

dans laquelle $X_2$, $X_4$, $X_5$, $R_2$, $R_3$, $R_4$ et $R_5$ ont les significations mentionnées précédemment,

* déprotection du composé de formule générale VIII par hydrogénation du groupe benzyle donnant lieu à la formation du composé de formule générale I dans laquelle $X_2$, $X_4$, $X_5$ , $R_2$ , $R_3$ , $R_4$ et $R_5$ ont les significations mentionnées précédemment.

**4.** A titre de médicament, le composé de formule générale I, selon l'une des revendications 1 et 2.

**5.** Utilisation des composés de formule générale I et de leurs sels physiologiquement acceptables, selon l'une des revendications 1 et 2, pour la préparation de médicaments destinés au traitement des maladies qui affectent le système nerveux central des mammifères.

**6.** Compositions pharmaceutiques caractérisées en ce qu'elles contiennent, outre un support pharmaceutiquement acceptable, au moins un composé de formule générale I ou un de ses sels physiologiquement acceptables, selon l'une des revendications 1 et 2.

## Claims

**1.** 2-[4-(4-azolylbutyl)-1-piperazinyl]pyrimidine derivatives, characterized in that they correspond to the general formula I,

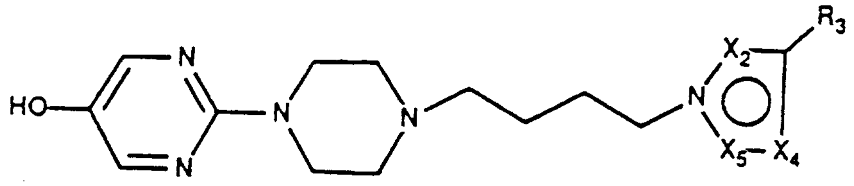

I

in which $X_2$ represents a nitrogen atom or a C-$R_2$ group, $X_4$ represents a nitrogen atom or a C-$R_4$ group, $X_5$ represents a nitrogen atom or a C-$R_5$ group, and $R_2$, $R_3$, $R_4$ and $R_5$, which are identical or different, represent a hydrogen atom, a halogen, a methyl radical, as well as their physiologically acceptable salts.

**2.** Compounds corresponding to the general formula I according to Claim 1, which are chosen from the following:

* 2-{4-[4-(4-chloropyrazol-1-yl)butyl]-1-piperazinyl}-5-hydroxypyrimidine-1-piperazinyl]pyrimidine
* 2-{4-[4-(4,5-dichloro-2-methylimidazol-1-yl)butyl]-1-piperazinyl}-5-hydroxypyrimidine
* 2-{4-[4-(4,5-dichloro-2 methylimidazol-1-yl)butyl]-1-piperazinyl}-5-hydroxypyrimidine hydrochloride.

**3.** Process for preparing the compounds of general formula I, according to Claims 1 and 2, characterized in that the following operations are carried out:

* oxidation of 5-benzyloxy-2-methylthiopyrimidine to yield the corresponding sulphone of formula V,

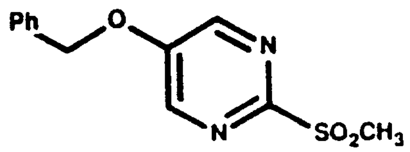

V

* replacement of the methylsulphonyl radical with a piperazine to yield the compound of formula VI,

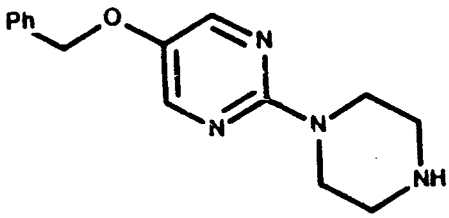

VI

* reaction of compound VI with dibromobutane to yield the corresponding spiro of formula VII,

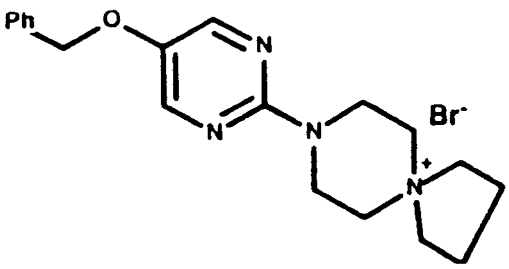

VII

* reaction of 8-(5-benzyloxy-2-pyrimidinyl)-8-aza-5-azoniaspiro[4.5]decane bromide with an azole of general formula IX

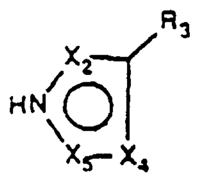

IX

in which $X_2$, $X_4$, $X_5$, $R_2$, $R_3$, $R_4$ and $R_5$ have the meanings indicated in Claim 1, to yield a compound of general formula VIII

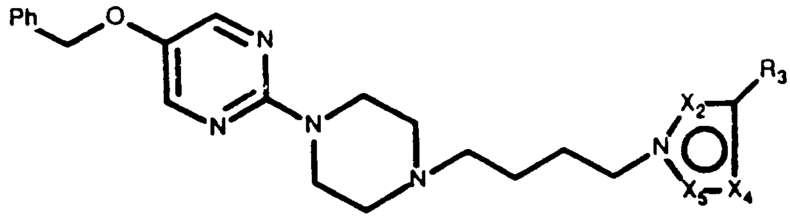

VIII

in which $X_2$, $X_4$, $X_5$, $R_2$, $R_3$, $R_4$ and $R_5$ have the meanings given earlier,

* deprotection of the compound of general formula VIII by hydrogenation of the benzyl group giving rise to the formation of the compound of general formula I in which $X_2$, $X_4$, $X_5$, $R_2$, $R_3$, $R_4$ and $R_5$ have the meanings given earlier.

**4.** Compound of general formula I, according to one of Claims 1 and 2, as medicinal product.

**5.** Use of the compounds of general formula I and their physiologically acceptable salts, according to one of Claims 1 and 2, for the preparation of medicinal products intended for the treatment of diseases which affect the central nervous system of mammalians.

**6.** Pharmaceutical compositions characterized in that they contain, in addition to a pharmaceutically acceptable carrier, at least one compound of general formula I or one of its physiologically acceptable salts, according to one of Claims 1 and 2.

**Patentansprüche**

**1.** Derivate von 2-[4-(4-Azolylbutyl)-1-piperazinyl]-pyrimidin, dadurch charakterisiert, daß sie der allgemeinen Formel I entsprechen

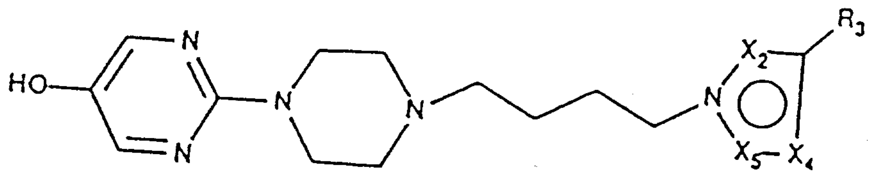

I

wobei $X_2$ ein Stickstoffatom oder eine Gruppe C-$R_2$ darstellt, $X_4$ ein Stickstoffatom oder eine Gruppe C-$R_4$ darstellt, $X_5$ ein Stickstoffatom oder eine Gruppe C-$R_5$ darstellt, und $R_2$, $R_3$, $R_4$ und $R_5$, identisch oder verschieden, ein Wasserstoffatom, ein Halogen, einen Methylrest darstellen und ihre physiologisch verträglichen Salze.

**2.** Der allgemeinen Formel I nach Anspruch 1 entsprechende Verbindungen, ausgewählt aus den folgenden:

* 2-{4-[4-(4-Chloropyrazol-1-yl-)butyl]-1-piperazinyl}-5-hydroxy-pyrimidin.
* 2-{4-[4-(4,5-Dichloro-2-methylimidazol-1-yl-)butyl]-1-piperazinyl}-5-hydroxy-pyrimidin.
* Hydrochlorid von 2-{4-[4-(4,5-Dichloro-2-methyl-imidazol-1-yl-)butyl]-1-piperazinyl}-5-hydroxy-pyrimidin.

**3.** Herstellungsverfahren für Verbindungen der allgemeinen Formel I nach den Ansprüchen 1 und 2, dadurch charakterisiert, daß man die folgenden Schritte durchführt:

* Oxidation von 5-Benzyloxy-2-methylthiopyrimidin zur Herstellung des entsprechenden Sulfons der Formel V

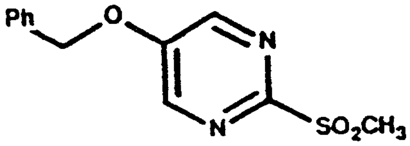

V

* Ersetzen des Methylsulfonylrestes durch ein Piperazin zur Herstellung der Verbindung der Formel VI,

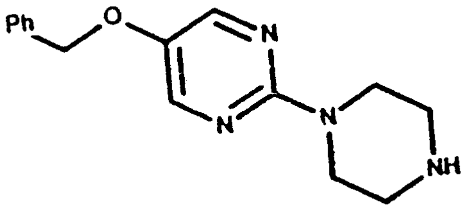

VI

* Reaktion der Verbindung VI mit Dibrombutan zur Herstellung der der Formel VII entsprechenden Spiroverbindung,

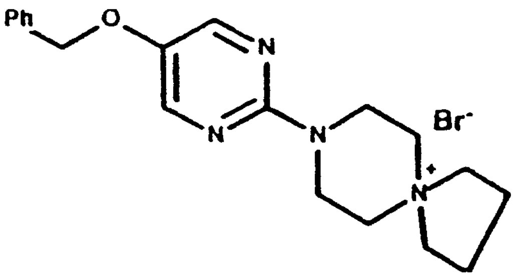

VII

* Reaktion von 8-(5-Benzyloxy-2-pyrimidinyl)-8-aza-5-azoniaspiro[4,5]decanbromid mit einem Azol der allgemeinen Formel IX

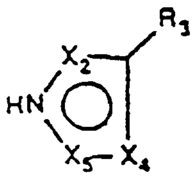

IX

wobei $X_2$, $X_4$, $X_5$, $R_2$, $R_3$, $R_4$ und $R_5$ die in Anspruch 1 angegebenen Bedeutungen haben, zur Herstellung einer Verbindung der allgmeinen Formel VIII

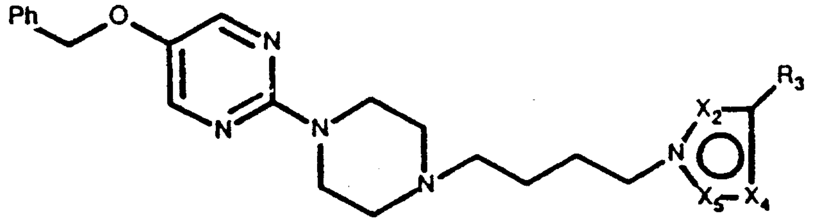

VIII

wobei $X_2$, $X_4$, $X_5$, $R_2$, $R_3$, $R_4$ und $R_5$ die zuvor erwähnten Bedeutungen aufweisen,

* Entschützen der Verbindung der allgemeinen Formel VIII durch Hydrierung der Benzylgruppe, was zur Bildung der Verbindung der allgemeinen Formel I führt, wobei $X_2$, $X_4$, $X_5$, $R_2$, $R_3$, $R_4$ und $R_5$ die zuvor angegebenen Bedeutungen aufweisen.

**4.** Verbindung der allgemeinen Formel I nach einem der Ansprüche 1 und 2 als Medikament.

**5.** Verwendung von Verbindungen der allgemeinen Formel I und ihrer physiologisch verträglichen Salze nach einem der Ansprüche 1 und 2 zur Herstellung von Medikamenten, die zur Behandlung von Erkrankungen bestimmt sind, die das zentrale Nervensystem von Säugern betreffen.

**6.** Pharmazeutische Zusammensetzungen, dadurch charakterisiert, daß sie neben einem pharmazeutisch verträglichen Träger mindestens eine Verbindung der allgemeinen Formel I oder eines ihrer physiologisch verträglichen Salze nach einem der Ansprüche 1 und 2 enthalten.